# EUROPEAN PATENT APPLICATION

(11) **EP 4 356 863 A1**
(43) Date of publication of application: **24.04.2024**
(21) Application number: 22841149.2
(22) Date of filing: 22.06.2022
(51) Int. Cl.: A61B 34/30, A61B 17/00

(54) **TRANSMISSION CONNECTION STRUCTURE FOR STERILE ADAPTER AND SURGICAL INSTRUMENT, AND INSTRUMENT DRIVE TRANSMISSION MECHANISM OF SURGICAL ROBOT**

(30) Priority: 14.07.2021 CN 202110797312
(71) Applicant: Cornerstone Technology (Shenzhen) Limited, Shenzhen, Guandong 518066 (CN)
(72) Inventor: ZHANG, Jianwei, Shenzhen, Guangdong 518066 (CN)
(74) Representative: Balder IP Law, S.L.
(86) International application number: PCT/CN2022/100536
(87) International publication number: WO 2023/284509

(57) **Abstract**

The present disclosure discloses a transmission and connection structure for a sterile adapter and a surgical instrument. The sterile adapter includes a sterile adapter transmission member, and the surgical instrument includes an instrument transmission member. The sterile adapter transmission member and the instrument transmission member each include a fitting surface, and the fitting surface includes a transmission groove fitting surface portion and a cylindrical fitting surface portion. The cylindrical fitting surface has a center on a rotation axis of the sterile adapter transmission member as well as a rotation axis of the instrument transmission member.

## Description

### CROSS REFERENCE TO RELATED APPLICATIONS

The present disclosure claims priority to Chinese Patent Application No. 202110797312.X, entitled "TRANSMISSION AND CONNECTION STRUCTURE FOR STERILE ADAPTER AND SURGICAL INSTRUMENT AND INSTRUMENT DRIVE TRANSMISSION MECHANISM FOR SURGICAL ROBOT," filed July 14, 2021, which is incorporated by reference herein in its entirety.

### TECHNICAL FIELD

The present disclosure relates to the technical field of medical devices, and more specifically to a transmission and connection structure for a sterile adapter and a surgical instrument and an instrument drive transmission mechanism for a surgical robot.

### BACKGROUND

Surgical robots have a large number of applications in clinical surgery as they help surgeons achieve precise positioning for surgery, have advantages such as reducing patient's wounds and thereby shortening post-operative recovery time, and have a stable operating platform capable of addressing situations such as tremors of surgeons.

A surgical instrument in the surgical robot typically has an end effector in the form of a surgical tool, such as forceps, scissors, clamps, etc., at one end of an elongated tube. In general, wires or ropes are employed to manipulate the end effector to pitch, yaw and grip.

A surgeon controls, at a console side, the instrument connected to a drive at a surgical side. In order to meet demands for different surgical instruments to be used during surgery, surgical instruments and the instrument drive are typically designed to be detachable for changing different surgical instruments during the surgery, and the surgical instruments are typically sterilized independently.

The instrument drive is typically designed to be non-sterilizable, and in order to ensure sterility during the surgery, a sterile adapter needs to be added between the instrument drive and the instrument during the surgery to isolate the non-sterilizable instrument drive and the sterilizable instrument during the surgery.

A back end of the surgical instrument is connected to an upper surface of the sterile adapter, the instrument drive is connected to a lower surface of the sterile adapter, and the instrument drive provides a driving force to the end effector of the surgical instrument through the sterile adapter for achieving pitch, yaw and grip of the end effector.

The lower surface of the sterile adapter is connected to an upper surface of the instrument drive, which are stable and undetached after connection, and meanwhile, the sterile adapter is also able to be easily and quickly unlocked and detached from the instrument drive when needed. The back end of the surgical instrument is connected to the upper surface of the sterile adapter, which are stable and undetached after connection, and meanwhile, the surgical instrument is also able to be easily and quickly unlocked and detached from the sterile adapter when needed.

During installation of the instrument drive, the sterile adapter and the surgical instrument, not only the three need to be engaged in place, but also an angle of an absolute position of the surgical instrument needs to be calibrated. In existing methods of installation of the three and calibration of the absolute position of the surgical instrument, two engaging members are provided on a transmission disc of the instrument drive, two recesses, fitted with the engaging members of the instrument drive, are provided underneath a transmission disc of the sterile adapter, two engaging members are provided on the transmission disc of the sterile adapter, and two recesses, fitted with the engaging members of the sterile adapter, are provided on a transmission disc of a back end of the instrument. During installation, fine tuning of the transmission disc of the sterile adapter and the transmission disc of the instrument drive is needed for multiple times until they engaged in place, and then the instrument drive drives the instrument drive transmission member and the transmission disc of the sterile adapter to rotate until the engaging members on the transmission disc of the sterile adapter are fitted with the recesses on the transmission disc of the surgical instrument. In this method, during the engagement of the transmission portion of the surgical instrument with the transmission portion of the sterile adapter and the engagement of the transmission portion of the sterile adapter with the transmission portion of the instrument drive, a user needs to be very meticulous during the installation and needs to adjust the angle several times in a vertical direction, left and right directions, and front and rear directions, in order to make a rotation axis of the transmission disc of the sterile adapter to be aligned with a rotation axis of the transmission disc of the instrument drive. This method also requires a motor of the instrument drive to drive the transmission disc of the instrument drive to rotate round and round for blind engagement, which needs several times of rotations to test and determine whether the engagement is in place, so as to complete the engagement.

### SUMMARY

A series of simplified concepts are introduced in the summary section, which are further described in details in the following embodiments. The summary section of the present disclosure is not intended to limit the key features and essential technical features of the claimed technical solution, let alone to determine the protection scope of the claimed technical solution.

A first aspect of embodiments of the present disclosure provides a transmission and connection structure for a sterile adapter and a surgical instrument to enable faster and more precise connection of transmission portions of the sterile adapter and the surgical instrument.

The first aspect of embodiments of the present disclosure provides a transmission and connection structure for a sterile adapter and a surgical instrument. The sterile adapter includes a sterile adapter transmission member, and the surgical instrument includes an instrument transmission member. The sterile adapter transmission member and the instrument transmission member each include a fitting surface, the fitting surface includes a transmission groove fitting surface portion and a cylindrical fitting surface portion, and the cylindrical fitting surface has a center on a rotation axis of the sterile adapter transmission member as well as a rotation axis of the instrument transmission member.

The transmission and connection structure for the sterile adapter and the surgical instrument provided in the first aspect of embodiments of the present disclosure enables the pre-connection of the sterile adapter transmission member and the instrument transmission member by the cylindrical fitting surface, and then the sterile adapter transmission member continues to rotate to find a unique fitting position of the sterile adapter transmission member and the instrument transmission member. The pre-connection enables the sterile adapter transmission member to be pre-aligned with the instrument transmission member, and the sterile adapter transmission member and the instrument transmission member then find a precise fitting position, which enables the sterile adapter transmission member and the instrument transmission member to be connected without inaccuracy.

In an embodiment, one of the sterile adapter transmission member and the instrument transmission member includes a cylindrical boss, and the other of the sterile adapter transmission member and the instrument transmission member includes a cylindrical sinking table. The cylindrical sinking table is sized to fit the cylindrical boss, and each of the cylindrical boss and the cylindrical sinking table has a center on the rotation axis of the sterile adapter transmission member as well as the rotation axis of the instrument transmission member.

In an embodiment, one of the sterile adapter transmission member and the instrument transmission member includes at least one transmission boss, and the other of the sterile adapter and the instrument transmission member includes at least one transmission groove. The at least one transmission boss and the at least one transmission boss are configured as fool-proofing structures. In this embodiment, the arrangement of the eccentric position of the transmission boss makes the sterile adapter transmission member and the instrument transmission member have a unique mounting position, which helps to calibrate the absolute position of each instrument transmission member on the surgical instrument, and this absolute position is used to find angles of rotations of the sterile adapter transmission member and the instrument transmission member at each movement of the surgical instrument.

In an embodiment, the sterile adapter transmission member includes a sterile adapter transmission member body and a cylindrical protrusion protruding from the sterile adapter transmission member body, and the instrument transmission member includes an instrument transmission member body and a cylindrical groove recessed into the instrument transmission member body. The cylindrical protrusion is configured to be inserted into and fitted with the cylindrical groove.

In an embodiment, the sterile adapter transmission member further includes a transmission boss protruding from the sterile adapter transmission member body, and the instrument transmission member further includes a transmission groove recessed into the instrument transmission member body. The transmission boss is configured to be inserted into and fitted with the transmission groove.

In an embodiment, the sterile adapter transmission member includes at least one transmission boss, and the at least one transmission boss is in an eccentric distribution with respect to a center of the cylinder.

In an embodiment, the sterile adapter transmission member includes at least two transmission bosses and at least two transmission grooves, and the at least two transmission bosses and at least two transmission grooves have a unique alignment position.

In an embodiment, the cylindrical protrusion has a height higher than a height of the transmission boss.

The cylindrical protrusion includes a guiding bevel along a circumference of the cylindrical protrusion at a top of the cylindrical protrusion.

In an embodiment, the sterile adapter includes an adapter body, and the adapter body defines a mounting hole for the sterile adapter transmission member; the sterile adapter transmission member is rotatably received in the mounting hole; and the sterile adapter transmission member is further provided with a positioning portion, the adapter body is further provided with a limiting portion in the mounting hole, and the limiting portion prevents the positioning portion from moving.

In an embodiment, the sterile adapter transmission member has a diameter at a lower end of the sterile adapter transmission member larger than a diameter of an upper end of the mounting hole.

A second aspect of embodiments of the present disclosure further provides an instrument transmission mechanism for a surgical robot, employing the transmission and connection structure for the sterile adapter and the surgical instrument in any of the above embodiments. The instrumentation drive mechanism for the surgical robot includes an instrument drive transmission member, a sterile adapter transmission member and an instrument transmission member, an instrument drive transmission member employing the transmission and connection structure for the sterile adapter and the surgical instrument in any of the above embodiments. A lower end of the sterile adapter is fitted with the instrument drive transmission member by means of a plurality of teeth evenly spaced and arranged on a surface of the sterile adapter transmission member engaging with a plurality of teeth evenly spaced and arranged on a surface of the instrument drive transmission member.

In this embodiment, the sterile adapter transmission member and the instrument drive transmission member are engaged by a plurality of teeth evenly spaced and arranged on the surfaces of both of them, which are able to fitted with each other at any angle. Meanwhile, the tooth-like structure is able to average torsion in all directions of the sterile adapter transmission member and the instrument drive transmission member, and has a longer service life.

A third aspect of embodiments of the present disclosure further provides an instrument transmission mechanism for a surgical robot, employing the transmission and connection structure for the sterile adapter and the surgical instrument in any of the above embodiments. The instrument drive mechanism for the surgical robot includes an instrument drive transmission member, a sterile adapter transmission member and an instrument transmission member, the sterile adapter transmission member and the instrument transmission member employ the transmission and connection structure for the sterile adapter and the surgical instrument in any of the above embodiments. A lower end of the sterile adapter transmission member is connected to the instrument drive transmission member by means of a transmission groove, and the sterile adapter transmission member is further connected to the instrument drive transmission member by means of a second cylindrical boss and a second cylindrical sinking table. The second cylindrical boss has a cylindrical fitting surface, the second cylindrical sinking table has a cylindrically recessed structure, and axes of the second cylindrical boss and the second cylindrical sinking table are located on the rotation axis of the sterile adapter transmission member as well as the rotation axis of the instrument drive transmission member.

In this embodiment, the sterile adapter transmission member is connected to the instrument drive instrument transmission member by means of the second cylindrical boss and the second cylindrical sinking table. The pre-connection of the cylindrical boss is performed before the two are precisely aligned, which enables faster and more precise engagement of the sterile adapter transmission member and the instrument drive transmission member.

In an embodiment, the transmission groove of the instrument drive transmission member and the transmission groove of the sterile adapter transmission member each has a fool-proofing structure. After the instrument drive transmission member and the sterile adapter transmission member have found a unique alignment position, the sterile adapter transmission member is then engaged with the instrument transmission member. The position of the sterile adapter transmission member and the instrument transmission member when they are connected is taken as the origin, and the angle of rotation of the sterile adapter transmission member at the time of the precise engagement of the sterile adapter transmission member and the instrument transmission member is used to calibrate an absolute position of the device drive member.

### BRIEF DESCRIPTION OF THE DRAWINGS

The following accompanying drawings of the present disclosure are hereby used as part of the present disclosure for understanding the present disclosure. Embodiments of the present disclosure and descriptions thereof are shown in the accompanying drawings to explain the principles of the present disclosure. In the accompanying drawings:
FIG. 1 is an assembly structure diagram of a mechanism of a drive transmission portion of a surgical instrument of a surgical robot in an embodiment of the present disclosure.
FIG. 2 is an exploded view of a mechanism of a drive transmission portion of a surgical instrument of a surgical robot in an embodiment of the present disclosure.
FIG. 3 is a top view of an instrument drive in an embodiment of the present disclosure.
FIG. 4 is a schematic diagram of an internal structure of an instrument drive in an embodiment of the present disclosure.
FIG. 5 is a schematic diagram of a connection structure for an instrument drive and a sterile adapter in an embodiment of the present disclosure.
FIG. 6 is a schematic structural diagram of an instrument drive transmission member of an instrument drive in an embodiment of the present disclosure.
FIG. 7 is a schematic structural diagram of a second tooth-like portion of a lower end portion of a sterile adapter transmission member of a sterile adapter in an embodiment of the present disclosure.
FIG. 8 is an exploded view of a connection structure for a sterile adapter and a transmission and connection structure of a surgical instrument in an embodiment of the present disclosure.
FIG. 9 is a disassembly structure diagram of a sterile adapter in an embodiment of the present disclosure.
FIG. 10 is a schematic diagram of a connection structure for an instrument drive and a sterile adapter in a second embodiment of the present disclosure.

### DETAILED DESCRIPTION OF THE EMBODIMENTS

In the following description, a number of specific details are given in order to provide a more thorough understanding of the present disclosure. However, it is apparent to those skilled in the art that embodiments of the present disclosure are able to be implemented without one or more of these details. In other examples, some technical features that are well known in the art are not described in order to avoid confusion with the embodiments of the present disclosure.

For a thorough understanding of the embodiments of the present disclosure, a detailed structure is presented in the following description. It is apparent that the implementation of the embodiments of the present disclosure is not limited to particular details familiar to those skilled in the art. Ordinal terms such as 'first' and 'second' cited in the present disclosure are merely identifiers and do not have any other meanings, such as a particular order, etc. Moreover, for example, the term 'first part' does not by itself imply the existence of a `second part', and the term `second part' does not by itself imply the existence of a `first part'. The terms 'top', 'bottom', 'front', `rear, 'left', 'right', and similar expressions are used in the present disclosure for illustrative purposes only and are not intended to be limiting.

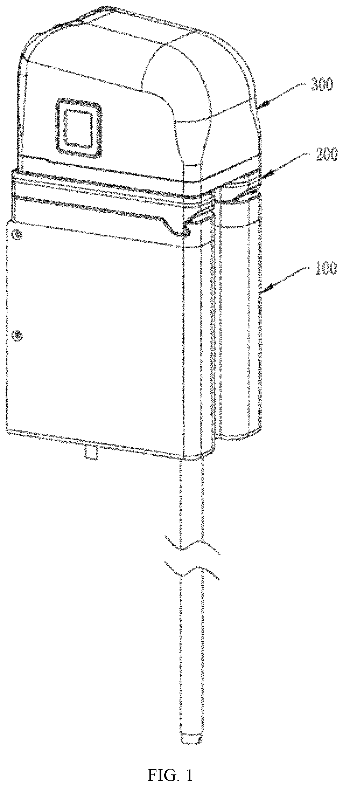

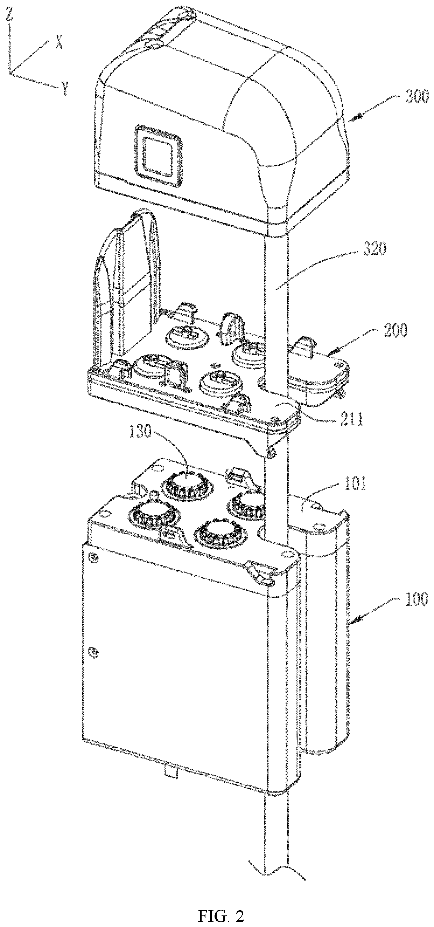

A surgical robot according to a first embodiment of the present disclosure includes a surgical trolley, a robotic arm, and an instrument drive 100, a sterile adapter 200 and a surgical instrument 300 that are mounted on a sliding arm of the robotic arm, as shown in FIG. 1 and FIG. 2.

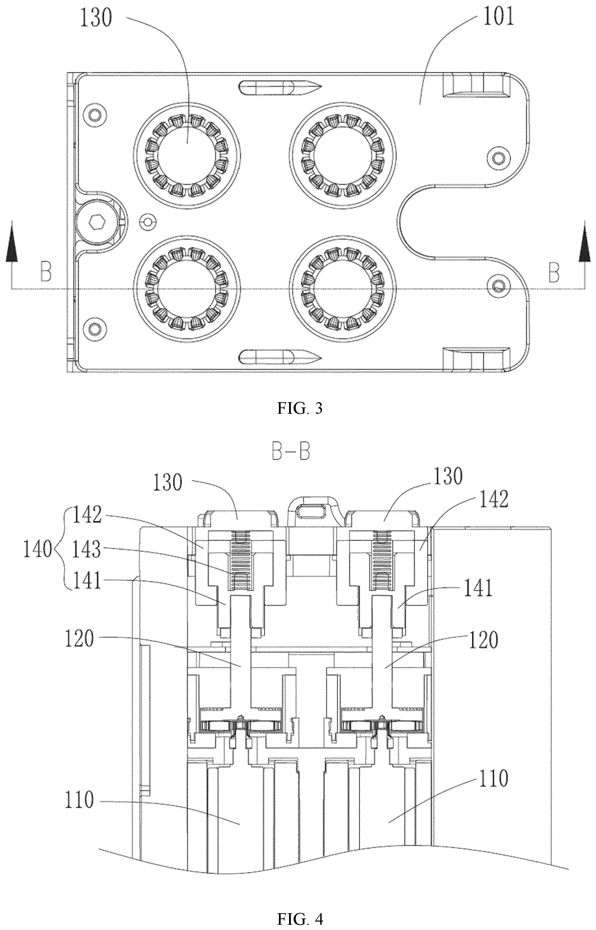

As shown in FIG. 3 and FIG. 4, a plurality of drive motors 110 are provided in the instrument drive 100, and each of the plurality of drive motors 110 is connected with an output shaft 120 and transmits drive forces to an effector of the surgical instrument through a transmission structure on a surface 101 of the instrument drive 100, a transmission structure of the sterile adapter 200, and a transmission structure of the surgical instrument 300, so as to achieve pitch, yaw, and grip of the effector.

In an embodiment, the instrument drive 100 includes an assembly surface 101 connected with the sterile adapter, and the assembly surface 101 of the instrument drive 100 defines mounting holes for mounting the instrument drive transmission members. Each of the instrument drive transmission members 130 is disposed within a respective mounting hole, and an upper surface of each instrument drive transmission member 130 protrudes from the assembly surface of the instrument drive. The number of instrument drive discs, which is the same as the number of output shafts, may be three, four, five, or more.

As shown in FIG. 4, a structure of each instrument drive transmission member 130 disposed within the instrument drive 100 is connected to a drive output shaft 120 by an elastic assembly 140. The elastic assembly 140 includes a first spring seat 141 secured to the output shaft, a second spring seat 142 secured to the instrument drive transmission member 130, and a spring 143 disposed between the first spring seat 141 and the instrument drive transmission member 130. The first spring seat 141 and the second spring seat 142 are engaged to seal the spring 143 in a cavity defined by the first spring seat 141, the second spring seat 142, and the instrument drive transmission member 130. In a natural state, due to pushing forces of the spring 143 applied to the two spring seats on both sides of the spring 143, the first spring seat 141 and the second spring seat 142 are able to be engaged with each other at edge positions of the first spring seat 141 and the second spring seat 142, and the instrument drive transmission member 130 is able to be lifted and protrude from the assembly surface 101 of the instrument drive.

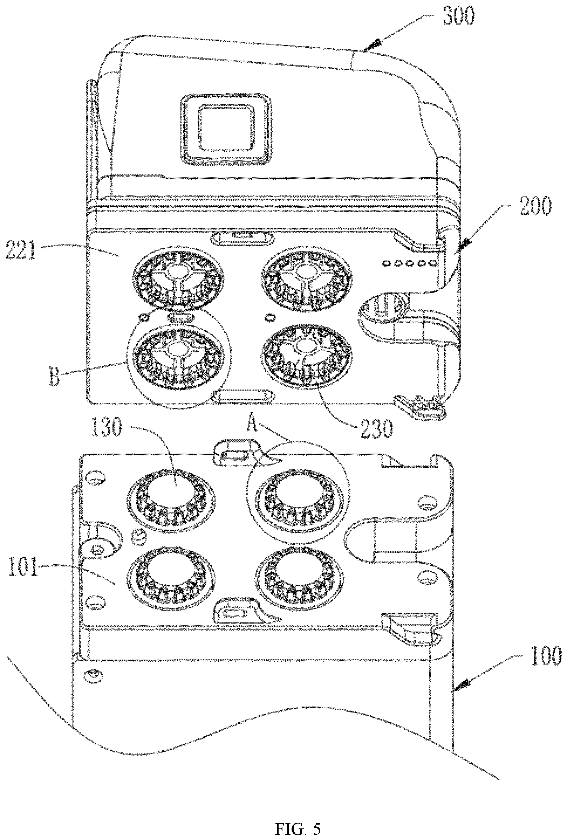

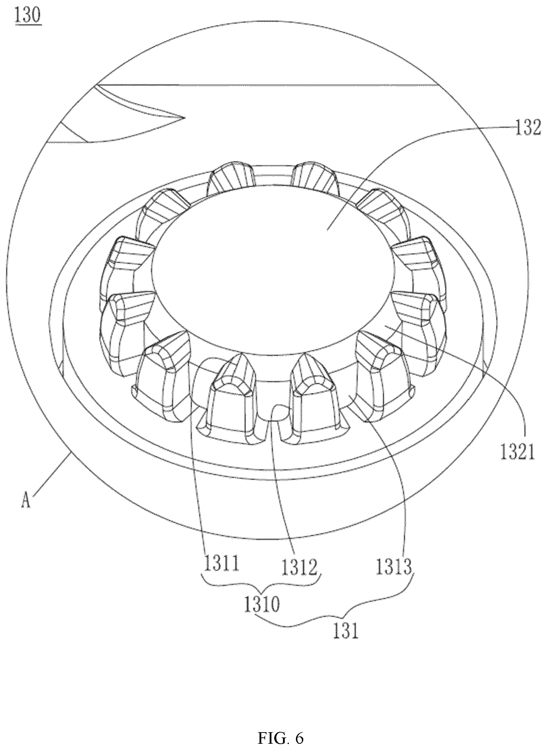

As shown in FIG. 5 and FIG. 6, each instrument drive transmission member 130 is substantially in the form of a cylindrical protrusion with a circular cross-section, a rotation axis of the instrument drive transmission member 130 is located at the center of the circular structure, and an upper end of the instrument drive transmission member 130 is in the form of a closed structure and has a first tooth-like portion 131 at the upper end or tip of the closed structure. The first tooth-like portion includes a plurality of first teeth 1310 that are distributed evenly in a radial direction of the instrument drive transmission member, i.e., the first teeth are arranged in a radial pattern. The number of the first teeth 1310 may be an odd number or an even number. A top of a respective first tooth, i.e., a tooth peak 1311 may be either sharp or rounded, in an effort to avoid having a platform at the tooth peak. Each first tooth 1310 has two meshing surfaces 1312 on two opposite sides of a lower end of the first tooth 1310, which may be planar in a vertical direction, or slightly inclined with respect to the vertical direction. Each first tooth 1310 is provided symmetrically or asymmetrically with respect to its own centerline, but the first tooth-like portion 131 is centrally symmetrical on the instrument drive transmission member 130. A first tooth slot 1313 is defined between two adjacent first teeth 1310. The instrument drive transmission member 130 includes a structure for guiding and positioning at the top, such as a disc surface portion 132 at the top of the instrument drive transmission member, which may be flush with or extend beyond a plane in which tooth peaks 1311 of the plurality of first teeth 1310 are located, and the disc surface portion 132 includes a bevel portion 1321 in a circumferential direction for guiding. Alternatively, in some embodiments, the top of the instrument drive transmission member does not have the disc surface portion, and a cylindrical member is provided at an axial position of the instrument drive transmission member, using the same guiding principle, with a bevel guide portion (not shown) provided at the top of the cylindrical member in a circumferential direction of the cylindrical member.

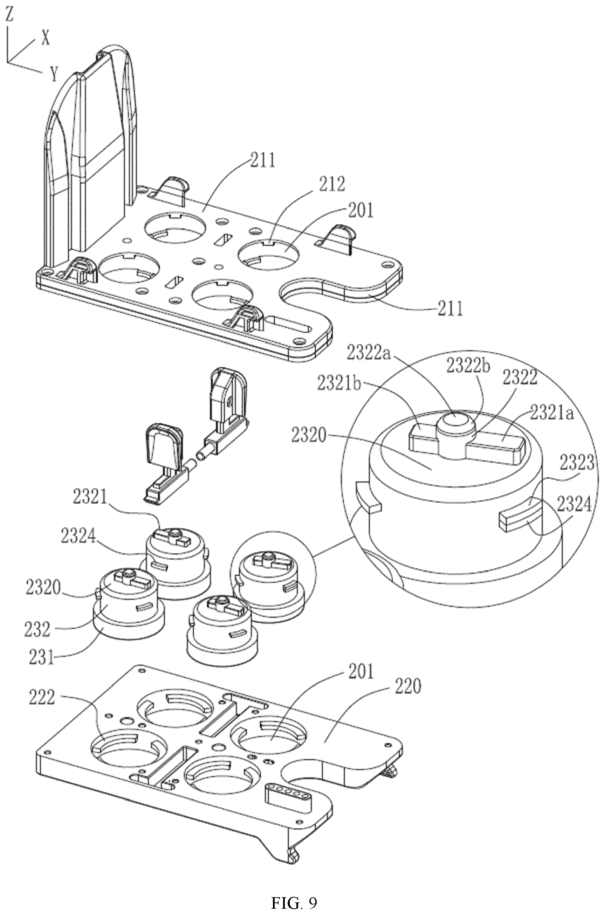

As shown in FIG. 5, the sterile adapter 200 has a lower surface 221 fitted with the assembly surface 101 of the instrument drive and an upper surface 211 fitted with a surgical instrument box at a back end of the surgical instrument. The sterile adapter 200 mainly includes an upper shell 210, a lower shell 220, and sterile adapter transmission members 230 penetrating the upper shell and the lower shell, as shown in FIG. 9. That is, each of the upper shell 210 and the lower shell 220 of the sterile adapter defines mounting holes 201 at same positions for the sterile adapter transmission members 230 to pass through. Each of the mounting holes 201 has a diameter that allows sufficient space for rotation of a respective sterile adapter transmission member 230. At the same time, each of the mounting holes 201 at the upper surface 211 of the sterile adapter has a diameter larger than a diameter of an upper end portion 232 of a respective sterile adapter transmission member and smaller than a diameter of a lower end portion 231 of the sterile adapter transmission member. In this way, the sterile adapter transmission member 230 is unable to be out of an upper end of the mounting hole 201 for the sterile adapter transmission member, and is unable to be detached from the mounting hole 201 for the sterile adapter transmission member during subsequent engagement of the sterile adapter and the surgical instrument. The number of the sterile adapter transmission members 230 is adapted to and the same as the number of the instrument drive transmission members 130 as well as the number of the driver output shafts 120.

Each sterile adapter transmission member 230 has a body structure having a cylindrical shape and a circular or annular cross-section. A rotation axis of the sterile adapter transmission member 230 is located at the center of the circular structure, i.e., the sterile adapter transmission member 230 and the instrument drive transmission member 130 are coaxial after the sterile adapter transmission member 230 is engaged with the instrument drive transmission member 130. The structure of the sterile adapter transmission member 230 may be a structure having one closed end spatially separating the instrument drive transmission member 130 and a surgical instrument transmission member 311.

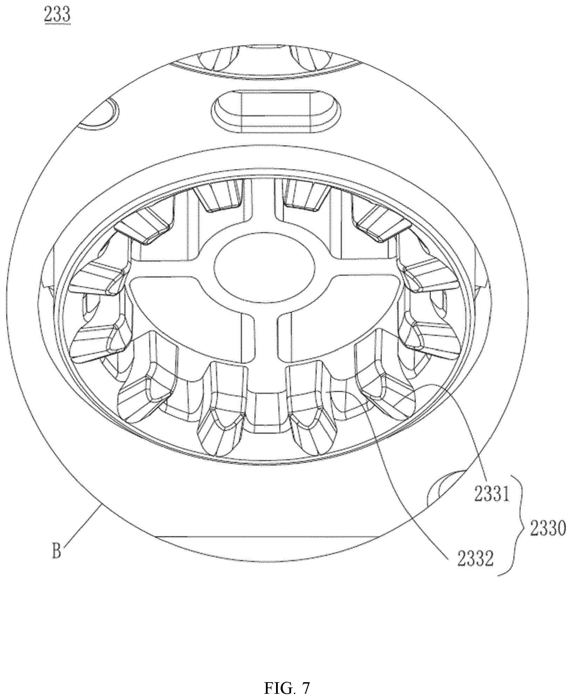

As shown in FIG. 7, the body structure of each sterile adapter transmission member has an upper end portion 232 for connecting a respective one of transmission members at the back end of the surgical instrument and a lower end portion 231 for connecting a respective instrument drive transmission member of the instrument drive. In one embodiment, each sterile adapter transmission member 230 has a cap shape with the lower end portion 231 having a surface portion or frame portion that fits with the instrument drive transmission member 130, and a second tooth-like portion 233 that engages the first tooth-like portion 131 of the instrument drive transmission member 130. The second tooth-like portion 233, similarly to the first tooth-like portion, includes a plurality of second teeth 2330 evenly distributed in a radial direction of the adapter, i.e., the second teeth are radially distributed. A tooth peak 2331 of each second tooth 2330 may be either sharp or rounded. Each second tooth 2330 has a meshing surface 2332 on two opposite sides, and the meshing surfaces 2332 on the two opposite sides of the second tooth 2330 are engageable with their opposite meshing surfaces 1312 of two adjacent first teeth 1310, i.e., the second tooth 2330 is insertable into a first tooth slot 1313 defined between the two adjacent first teeth 1310. In the illustrated embodiments, the second tooth-like portion is provided at an inner periphery of the body of the sterile adapter transmission member 230 and the first tooth-like portion is provided at an outer periphery of the body of the instrument drive transmission member 130, and a state in which the second tooth-like portion 233 is combined with the first tooth-like portion 131 is like that the second tooth-like portion forms an engaging cover on the first tooth-like portion. In some embodiments, the second tooth-like portion is provided at the outer periphery of the body of the adapter and the first tooth-like portion is provided at the inner periphery of the body of the instrument drive transmission member, such that the second tooth-like portion is combined with the first tooth-like portion as if the second tooth-like portion is inserted in the first tooth-like portion. Of course, in some embodiments, the first tooth-like portion and the second tooth-like portion have identical shapes, the tooth-like portions are all exposed structures, and the first tooth-like portion and the second tooth-like portion are coupled to form a complete cylinder. All transmission structures using the same principle of toothed engagement are considered to be equivalent solutions in the embodiments of the present disclosure.

When the sterile adapter 200 is mounted and fitted with the instrument drive 100, each sterile adapter transmission member 230 is connected to a respective instrument drive transmission member 130 at any angle through the engagement of the second tooth-like portion 233 with the first tooth-like portion 131 without pre-alignment or alignment. Meanwhile, the toothed structure is capable of averaging torsion in all directions of the sterile adapter transmission member and the instrument drive transmission member for a longer service life.

Connection of a transmission mechanism of the sterile adapter and the surgical instrument is as follows.

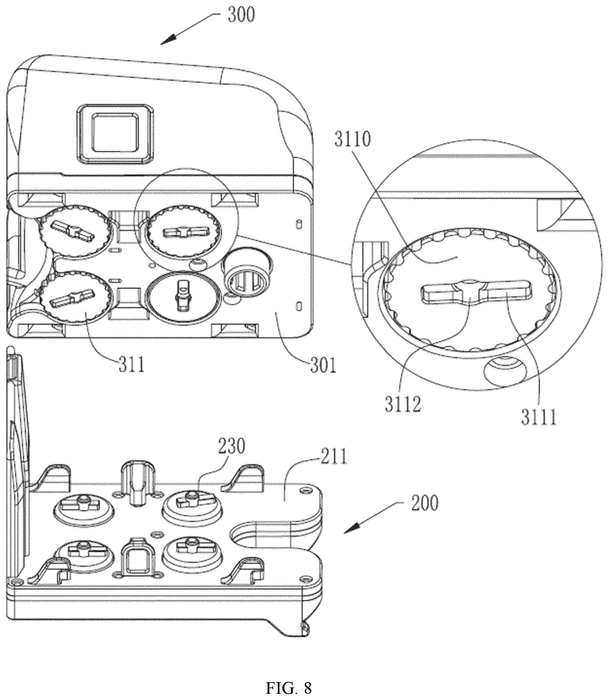

The surgical instrument 300, as shown in FIG. 2 and FIG. 8, includes a surgical instrument box 310 disposed at the back end of the surgical instrument, a surgical instrument disposed at a front end of the surgical instrument, an instrument shaft 320 connecting the front end and the back end, and a transmission mechanism within the instrument shaft. A lower surface 301 of the surgical instrument box is fitted with an upper surface 211 of the sterile adapter. The surgical instrument box includes a mounting seat at a lower end, and the mounting seat includes a cylinder structure for connecting the transmission mechanism, such as the steel wire. Each cylindrical protrusion has a lower end fixedly connected with the instrument transmission member 311, and the number of instrument transmission members is the same as the number of the sterile adapter transmission members as well as the number of the instrument drive discs.

As shown in FIG. 9, the upper end portion 232 of the sterile adapter transmission member has an end surface 2320 that is capable of closely abutting an end surface 3110 of the instrument transmission member of the surgical instrument box 310, and the sterile adapter transmission member includes a transmission boss 2321 protruding from the upper end portion 232, which is engaged with the surgical instrument transmission member 311 to provide transmission torque. In one embodiment, at least one transmission boss 2321 having an eccentric structure may be provided on the same sterile adapter transmission member 230, i.e., each of the at least one transmission boss 2321 has a non-axis symmetric, non-planar symmetric or non-centrally symmetric structure. In short, whether one or more transmission bosses are provided, the transmission boss is configured as a fool-proofing structure on the sterile adapter transmission member such that there is only a unique alignment position on both the sterile adapter transmission member as well as on the instrument transmission member when they are engaged.

Each instrument transmission member 311 has the end surface 3110 engaged with the sterile adapter transmission member as well as substantially matching the size of the end surface 2320 of the sterile adapter transmission member, and has a rotation axis the same as the rotation axis of the sterile adapter transmission member. The structure and number of the instrument transmission members 311 on the surgical instrument box are both adapted to the sterile adapter transmission members 230, and each instrument transmission member defines a transmission groove 3111 for fitting with a respective transmission boss 2321. The transmission groove 3111 is formed as an inwardly recessed structure on the instrument transmission member 311, i.e., the transmission groove 3111 is inwardly recessed from the end surface of the instrument transmission member 311. A structure of the transmission groove 3111 is adapted to a shape of the transmission boss 2321, or at least adapted to the transmission boss 2321 in a direction of transmitting power (i.e., a direction of rotation around the axial center), such as contact points and contact surfaces for forming a torque in a direction of rotation of the sterile adapter transmission member 230 and the instrument transmission member 311 are adapted. On the premise that the engagement of the transmission boss 2321 and the transmission groove 3111 has a certain engagement depth, a depth of the transmission groove 3111 may not be strictly required.

In the illustrated embodiments, the sterile adapter transmission member is provided with two transmission bosses 2321 including a larger transmission boss 2321a and a smaller transmission boss 2321b, which extend in opposite directions on the end surface 2320 of the sterile adapter transmission member 230 and have an end not at the same distance from a center position. Accordingly, the instrument transmission member has two transmission grooves 3111, one adapted to the larger transmission boss and the other adapted to the smaller transmission boss. The setting of the two transmission bosses and the transmission grooves in this embodiment equalizes the transmission of torque, and at the same time, their asymmetric structures make the adapter transmission member and the instrument transmission member at the back end of the instrument only have a unique alignment position when they are connected.

In one embodiment, each sterile adapter transmission member 230 includes a cylindrical protrusion 2322 protruding from the end surface 2320, which is a cylindrical boss having a rotation axis the same as the rotation axis of the sterile adapter transmission member 230, i.e., the cylindrical protrusion 2322 is located at the center of the sterile adapter transmission member 230. Accordingly, a respective instrument transmission member 311 defines a cylindrical groove 3112, and the cylindrical protrusion 2322 is able to be inserted in the cylindrical groove 3112. In one embodiment, a height of the cylindrical protrusion 2322 is higher than a height of the transmission boss 2321, i.e., a distance between an end surface of the cylindrical protrusion 2322 and the end surface 2320 of the sterile adapter transmission member is greater than a distance between an end surface of the transmission boss 2321 and the end surface 2320 of the sterile adapter transmission member. In this way, the cylindrical groove 3112 is pre-connected with the cylindrical protrusion 2322 before the mounting of the instrument transmission member 311.

In some embodiments, when each cylindrical protrusion 2322 has a physical connection with a respective transmission boss 2321, i.e., the cylindrical protrusion 2322 is integral with the transmission boss 2321 without void space as shown in the figures, a diameter of the cylindrical protrusion 2322 is greater than a width of the transmission boss 2321. Meanwhile, a diameter of the cylindrical groove for fitting with the cylindrical boss should be greater than a width of the transmission groove in the instrument transmission member at the rear end of the instrument, i.e., a width between two torque surfaces of the transmission boss 2321 is less than a diameter of the cylindrical protrusion 2322 as shown in the figures, which makes the pre-connecting effective.

In some embodiments, each cylindrical protrusion 2322 further has a guiding bevel 2322a along a circumference the cylindrical protrusion at the top. That is, a diameter of an end surface 2322b of the cylindrical protrusion is smaller than a diameter of a lower portion of the cylindrical protrusion 2322, and a diameter of the cylindrical protrusion in a certain portion is progressively increasing from the end surface 2322b of the cylindrical protrusion to the end surface of the sterile adapter transmission member. In this way, the cylindrical protrusion forms a portion of non-cylindrical protrusion having a bevel starting from the top. This portion of the cylindrical protrusion facilitates faster inserting of the cylindrical protrusion into the cylindrical groove when the cylindrical protrusion is pre-connected with the cylindrical groove.

In some embodiments, the cylindrical protrusion is also able to be provided on the instrument transmission member of the surgical instrument, and a corresponding cylindrical groove is provided on the sterile adapter transmission member of the sterile adapter. Similarly, the transmission boss is also able to be provided on the instrument transmission member of the surgical instrument, and a corresponding transmission groove is provided on the sterile adapter transmission member. The same technical effect as in the above embodiments is able to be achieved.

Furthermore, in one embodiment, each sterile adapter transmission member 230 further includes at least one positioning portion 2323 disposed at an outer periphery of the upper end portion 232 of the sterile adapter transmission member, and at least one limiting portion 212 inside the mounting hole 201 for the sterile adapter transmission member. The at least one limiting portion 212 is disposed substantially at a position of the upper shell 210 of the sterile adapter adjacent to the upper surface 211 of the sterile adapter. When the at least one limiting portion 212 is located at substantially the same level as the at least one positioning portion 232, the sterile adapter transmission member rotates so as to make the at least one positioning portion 232 rotate to the at least one limiting portion 212. The at least one limiting portion prevents the at least one positioning portion from rotating such that the sterile adapter transmission member stops rotating, and such a position is able to be used as an original position of the sterile adapter.

Each sterile adapter transmission member 230 further includes at least one stop portion 2324 disposed at the outer periphery of the sterile adapter, and the sterile adapter transmission member includes at least one block portion 222 provided at the lower portion of the mounting hole 201 and configured to prevent the at least one stop portion 2324 from moving, so that a structure of the sterile adapter is complete when it is not mounted, and the sterile adapter transmission member is not prone to fall off.

In one embodiment, the at least one positioning portion 2323 is higher than the at least one stop portion 2324 on the sterile adapter transmission member, the at least one limiting portion 212 at the upper portion of the mounting hole 201 for the sterile adapter transmission member is configured to prevent the at least one positioning portion 2323 from moving, and the at least one block portion 222 provided at the lower portion of the mounting hole 201 is configured to prevent the at least one stop portion 2311 from moving.

In one embodiment, one of the at least one stop portion 2324 is integral with the portioning structure 2323.

In one embodiment, in the mounting process of the sterile adapter and the instrument, the sterile adapter is mounted to the instrument drive in any orientation, the output shaft of the instrument drive jacks up the instrument drive transmission member and the sterile adapter transmission member, so that the limiting portion on the sterile adapter and the positioning portion on the sterile adapter are at a substantial level, and then a motor of the instrument drive rotates to drive the instrument drive transmission member and the sterile adapter transmission member to rotate so that the positioning portion is abutted against the limiting portion. Such a position of the sterile adapter transmission member at this time is able to be labeled as an original position of the sterile adapter transmission member.

In the mounting process of the surgical instrument and the sterile adapter, the back end of the surgical instrument is mounted from up to down, and the cylindrical protrusion of the sterile adapter transmission member is inserted into the cylindrical groove of the surgical instrument transmission member to achieve pre-connecting when the surgical instrument transmission member is connected with the sterile adapter. At this time, the transmission bosses of the sterile adapter transmission member and the transmission grooves of the surgical instrument transmission member are not aligned such that the transmission bosses of the sterile adapter transmission member abut against a surface of the surgical instrument transmission member, and the transmission bosses on the sterile adapter transmission member are pressed downwardly, which causes the at least one the positioning portion to disengage from the at least one limiting portion. Then, the motor of the instrument drive rotates to drive the sterile adapter transmission member to rotate until the transmission bosses on the sterile adapter transmission member are aligned and connected with the transmission grooves of the surgical instrument transmission member to complete the mounting. At this time, an angle at which the sterile adapter transmission member rotates with respect to the original position is an absolute position of the surgical instrument, and this absolute position is used to determine various angles of action of the surgical instrument.

A surgical robot according to a second embodiment of the present disclosure differs from the surgical robot in the first embodiment in the connection structure between the instrument drive transmission member and the sterile adapter transmission member. In the second embodiment, the instrument drive transmission member and the sterile adapter are connected by means of transmission bosses of different sizes and a pre-connecting structure.

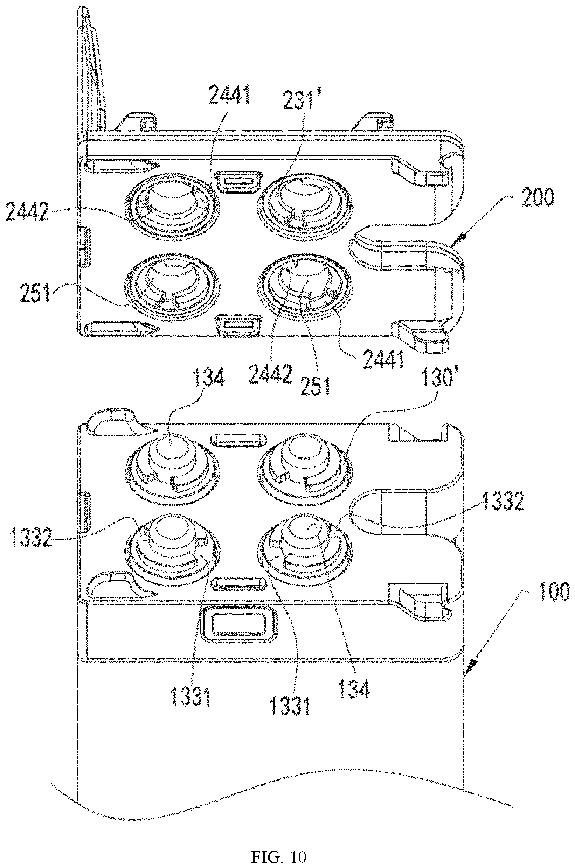

FIG. 10 shows a connection structure for the instrument drive and the sterile adapter in a second embodiment. Each of a plurality of sterile adapter transmission members 231' includes two second transmission bosses of different sizes at a lower end, i.e., a larger second transmission boss 2411 and a smaller second transmission boss 2412. Accordingly, each of a plurality of instrument drive transmission members 130' defines a larger second transmission groove 1331 and a smaller second transmission groove. The second transmission bosses are configured as fool-proofing structures such that each sterile adapter transmission member and a respective instrument drive transmission member have a unique alignment position. Meanwhile, a second cylindrical boss 134 is further provided on each instrument drive transmission member and a second cylindrical sinking table 251 is further defined on each sterile adapter. The second cylindrical boss has a cylindrical fitting surface, and the second cylindrical sinking table 251 has a cylindrically recessed structure. The second cylindrical boss 134 is connected with the second cylindrical sinking table 251 so that axes of rotation of the second cylindrical boss 134 and the second cylindrical sinking table 251 are coaxial with axes of rotation of the sterile adapter transmission member and the instrument drive transmission member. The second cylindrical boss and the second cylindrical sinking table are provided such that the second cylindrical boss 134 is able to be pre-connected with the second cylindrical sinking table 251 before the sterile adapter transmission member and the instrument drive transmission member are connected.

All other structures in the second embodiment are the same as those in the first embodiment, such as positioning and stop structures of the sterile adapter transmission member and the sterile adapter, and the transmission bosses of different sizes as well as the pre-connecting structure connected between the sterile adapter transmission member and the surgical instrument drive transmission member, which are not repeated herein.

In this embodiment, the instrument drive transmission member is pre-connected with the sterile adapter transmission member first, i.e., the second cylindrical boss of the instrument drive transmission member is pre-connected with the second cylindrical sinking table of the sterile adapter, and then the instrument transmission member is pre-connected with the sterile adapter transmission member, i.e., the cylindrical protrusion on the sterile adapter transmission member is pre-connected with the cylindrical groove on the instrument transmission member. Then the motor of the instrument drive rotates to drive the instrument drive transmission member to rotate, and the second cylindrical boss and the second cylindrical sinking table that are pre-connected between the instrument drive transmission member and the sterile adapter transmission member drive the sterile adapter transmission member to rotate due to mutual friction. When the positioning portion inside the mounting hole for the sterile adapter transmission member prevents the positioning portion on the sterile adapter transmission member from moving, the sterile adapter transmission member stops rotating, and the instrument drive transmission member continues to rotate to find a unique alignment position of the sterile adapter transmission member, i.e., the second groove of the instrument drive transmission member is located just below the second transmission boss of the sterile adapter. The instrument drive transmission member is connected with the sterile adapter, and a position of the sterile adapter transmission member and the instrument drive transmission member at this time is taken as the original position. The instrument drive transmission member and the sterile adapter transmission member continue to rotate until the sterile adapter transmission member is precisely connected with the instrument transmission member, and the angle at which the sterile adapter transmission member rotates with respect to the original position is the absolute position of the instrument transmission member.

Unless otherwise defined, technical and scientific terms used herein have the same meaning as commonly understood by those skilled in the art. Terms used herein are intended to describe specific embodiments only and are not intended to limit the present disclosure. Terms such as 'disposed' as they appear herein may indicate either a component attached directly to another component or a component attached to another component via an intermediate member. Features described herein in one embodiment may be applied to another embodiment, either alone or in combination with other features, unless the feature is not applicable in that other embodiment or is otherwise described.

The present disclosure has been described by the above embodiments, which are used for purposes of example and illustration only and are not intended to limit the present disclosure to the scope of the described embodiments. More variations and modifications may be made in accordance with the teachings of the present disclosure, all of which fall within the protection scope claimed in the present disclosure.

## Claims

1. A transmission and connection structure for a sterile adapter and a surgical instrument, wherein the sterile adapter includes a sterile adapter transmission member, and the surgical instrument includes an instrument transmission member; and wherein:
the sterile adapter transmission member and the instrument transmission member each include a fitting surface, wherein the fitting surface includes a transmission groove fitting surface portion and a cylindrical fitting surface portion, and wherein the cylindrical fitting surface has a center on a rotation axis of the sterile adapter transmission member as well as a rotation axis of the instrument transmission member.

2. The transmission and connection structure according to claim 1, wherein one of the sterile adapter transmission member and the instrument transmission member includes a cylindrical boss, and the other of the sterile adapter transmission member and the instrument transmission member includes a cylindrical sinking table; and wherein the cylindrical sinking table is sized to fit the cylindrical boss, and each of the cylindrical boss and the cylindrical sinking table has a center on the rotation axis of the sterile adapter transmission member as well as the rotation axis of the instrument transmission member.

3. The transmission and connection structure according to claim 1, wherein one of the sterile adapter transmission member and the instrument transmission member includes at least one transmission boss, and the other of the sterile adapter and the instrument transmission member includes at least one transmission groove; and wherein the at least one transmission boss and the at least one transmission boss are configured as fool-proofing structures.

4. The transmission and connection structure according to any one of claims 1 to 3, wherein the sterile adapter transmission member includes a sterile adapter transmission member body and a cylindrical protrusion protruding from the sterile adapter transmission member body, and the instrument transmission member includes an instrument transmission member body and a cylindrical groove recessed into the instrument transmission member body; and wherein the cylindrical protrusion is configured to be inserted into and fitted with the cylindrical groove, and the cylindrical protrusion and the cylindrical groove each include a cylindrical fitting surface.

5. The transmission and connection structure according to claim 4, wherein the sterile adapter transmission member further includes a transmission boss protruding from the sterile adapter transmission member body, and the instrument transmission member further includes a transmission groove recessed into the instrument transmission member body; and wherein the transmission boss is configured to be inserted into and fitted with the transmission groove.

6. The transmission and connection structure according to claim 5, wherein the sterile adapter transmission member includes at least one transmission boss, and the at least one transmission boss is in an eccentric distribution with respect to a center of the cylinder.

7. The transmission and connection structure according to claim 5, wherein the sterile adapter transmission member includes at least two transmission bosses and at least two transmission grooves, and the at least two transmission bosses and at least two transmission grooves have a unique alignment position.

8. The transmission and connection structure according to any one of claims 5 to 7, wherein the cylindrical protrusion has a height higher than a height of the transmission boss.

9. The transmission and connection structure according to any one of claims 4 to 8, wherein the cylindrical protrusion includes a guiding bevel extending along a circumference of the cylindrical protrusion at a top of the cylindrical protrusion.

10. The transmission and connection structure according to any one of claims 1 to 9, wherein the sterile adapter includes an adapter body, and the adapter body defines a mounting hole for the sterile adapter transmission member; wherein the sterile adapter transmission member is rotatably received in the mounting hole; and wherein the sterile adapter transmission member is further provided with a positioning portion, the adapter body is further provided with a limiting portion in the mounting hole, and the limiting portion prevents the positioning portion from moving.

11. The transmission and connection structure according to claim 10, wherein the sterile adapter transmission member has a diameter at a lower end of the sterile adapter transmission member larger than a diameter of an upper end of the mounting hole.

12. An instrument drive transmission mechanism for a surgical robot, comprising:
an instrument drive transmission member, a sterile adapter transmission member and an instrument transmission member, wherein the sterile adapter transmission member and the instrument transmission member employ the transmission and connection structure for the sterile adapter and the surgical instrument according to any one of claims 1 to 11, and a lower end of the sterile adapter is fitted with the instrument drive transmission member by means of a plurality of teeth evenly spaced and arranged on a surface of the sterile adapter transmission member engaging with a plurality of teeth evenly spaced and arranged on a surface of the instrument drive transmission member.

13. An instrument drive transmission mechanism for a surgical robot comprising: an instrument drive transmission member, a sterile adapter transmission member and an instrument transmission member, wherein the sterile adapter transmission member and the instrument transmission member employ the transmission and connection structure for the sterile adapter and the surgical instrument according to any one of claims 1 to 11, a lower end of the sterile adapter transmission member is connected to the instrument drive transmission member by means of a transmission groove, and the sterile adapter transmission member is further connected to the instrument drive transmission member by means of a second cylindrical boss and a second cylindrical sinking table; and wherein the second cylindrical boss has a cylindrical fitting surface, the second cylindrical sinking table has a cylindrically recessed structure, and axes of the second cylindrical boss and the second cylindrical sinking table are located on the rotation axis of the sterile adapter transmission member as well as the rotation axis of the instrument drive transmission member.

14. The instrument drive transmission mechanism for the surgical robot according to claim 13, wherein the transmission groove for connection of the instrument drive transmission member and the sterile adapter transmission member has a fool-proofing structure.
